# EUROPÄISCHE PATENTANMELDUNG

(11) **EP 3 797 699 A1**
(43) Veröffentlichungstag der Anmeldung: **31.03.2021**
(21) Anmeldenummer: 19200518.9
(22) Anmeldetag: 30.09.2019
(51) Int. Cl.: A61B 6/03, A61B 5/00, A61B 5/055, A61B 6/00, G01R 33/48, A61B 6/04

(54) **VERFAHREN ZUR ERSTELLUNG VON BILDDATEN MITTELS EINES KOMBINIERTEN BILDGEBUNGSGERÄTS**

(71) Anmelder: Siemens Healthcare GmbH, 91052 Erlangen (DE)
(72) Erfinder: Fenchel, Matthias, 91054 Erlangen (DE)

(57) **Zusammenfassung**

Die Erfindung betrifft ein Verfahren zur Erstellung von Bilddaten eines Untersuchungsobjektes unter Verwendung eines kombinierten Bildgebungsgeräts, welches ein erstes Bildgebungsverfahren und ein zweites Bildgebungsverfahren aufweist, wobei das erste Bildgebungsverfahren eine strukturelle Bildgebung aufweist und das zweite Bildgebungsverfahren eine funktionelle Bildgebung aufweist. Die Schritte des Verfahrens umfassen eine Aufnahme einer ersten Übersichtsmessung mittels des ersten Bildgebungsverfahrens und einer zweiten Übersichtsmessung mittels des zweiten Bildgebungsverfahrens, wobei sich die erste Übersichtsmessung mit der zweiten Übersichtsmessung zeitlich überschneidet, ein Erstellen von ersten Übersichtsbilddaten basierend auf der ersten Übersichtsmessung und von zweiten Übersichtsbilddaten basierend auf der zweiten Übersichtsmessung, eine Planung einer Messung des ersten Bildgebungsverfahrens und des zweiten Bildgebungsverfahrens auf Basis der ersten Übersichtsbilddaten und der zweiten Übersichtsbilddaten, eine Durchführung der geplanten Messung des ersten Bildgebungsverfahrens und des zweiten Bildgebungsverfahrens sowie ein Erstellen von Bilddaten des Untersuchungsobjektes auf Basis der Messung des ersten Bildgebungsverfahrens und des zweiten Bildgebungsverfahrens.

## Beschreibung

Die Erfindung betrifft ein Verfahren zur Erstellung von Bilddaten unter Verwendung eines kombinierten Bildgebungsgeräts, eine Messeinheit, ein kombiniertes Bildgebungsgerät und ein Computerprogrammprodukt.

In der medizinischen Diagnostik, insbesondere der bildgebenden Diagnostik, werden Untersuchungen häufig mittels kombinierten Bildgebungsgeräten durchgeführt, welche mehr als ein Bildgebungsverfahren, typischerweise zwei Bildgebungsverfahren, aufweisen. In diesen Untersuchungen werden medizinische Bilddaten von einem Untersuchungsobjekt mittels der mehreren, insbesondere zwei, Bildgebungsverfahren, insbesondere gleichzeitig, erfasst. Die Bilddaten werden dabei mit unterschiedlichen medizintechnischen bildgebenden Geräten, wie z. B. Magnetresonanztomographen, Positronen-Emissionstomographen oder Einzel-Photonen-Emissionscomputertomographen, akquiriert. Besonders sinnvoll ist es dabei Bildgebungsverfahren zu kombinieren, die sowohl strukturelle Informationen des Untersuchungsobjektes (z. B. durch Magnetresonanztomographie), als auch funktionelle Informationen des Untersuchungsobjektes (z. B. durch Positronen-Emissionstomographie) erfassen. Die Beurteilung der medizinischen Bilddaten wird damit einer fachkundigen Person erleichtert, da der fachkundigen Person die Bilddaten beider Bildgebungsverfahren zur Verfügung stehen.

Der Messung mit einem kombinierten Bildgebungsgerät geht üblicherweise eine Planung der Messung voraus. Diese kann beispielsweise eine Planung des Bildgebungsbereiches ("field of view") der Messung im Untersuchungsobjekt oder eine Planung weiterer Bildgebungsparameter umfassen. Für diese Planung werden häufig sogenannte Übersichtsbilddaten ("scouts") auf Basis der Magnetresonanztomographie herangezogen, welche in einer geringen Zeit aufgenommen werden können, aber eine relativ grobe Auflösung und eine unvollkommene anatomische Darstellung aufweisen. Insbesondere können bei diesen Übersichtsbilddaten auch relevante anatomische oder pathologische Strukturen fehlen, da diese durch die Magnetresonanztomographie nicht adäquat aufgelöst werden. Die Planung der Messung kann somit nicht optimal durchgeführt werden.

Es ist eine Aufgabe der vorliegenden Erfindung, eine zeiteffiziente Planung der Messung eines kombinierten medizinischen Bildgebungsgeräts zu ermöglichen. Die Aufgabe wird durch die Merkmale der unabhängigen Ansprüche gelöst. Vorteilhafte Ausgestaltungen sind in den Unteransprüchen beschrieben.

Das erfindungsgemäße Verfahren zur Erstellung von Bilddaten eines Untersuchungsobjekts unter Verwendung eines kombinierten Bildgebungsgeräts, welches ein erstes Bildgebungsverfahren und ein zweites Bildgebungsverfahren aufweist, umfasst die folgenden Verfahrensschritte:
Aufnahme einer ersten Übersichtsmessung mittels eines ersten Bildgebungsverfahrens und einer zweiten Übersichtsmessung mittels eines zweiten Bildgebungsverfahrens, wobei sich die erste Übersichtsmessung zeitlich mit der zweiten Übersichtsmessung überschneidet. Das erste Bildgebungsverfahren weist dabei eine strukturelle Bildgebung mittels eines Magnetresonanzgerätes auf. Das zweite Bildgebungsverfahren weist eine funktionelle Bildgebung, wie z. B. die Positronen-Emissionstomographie (PET) oder die Einzel-Photonen-Emissionscomputertomographie (SPECT) auf.

Die Messdaten der ersten Übersichtsmessung mit dem Magnetresonanztomographen liegen typischerweise in einem k-Raum vor. Zur Aufnahme des k-Raums können die Magnetisierung von Protonen im Untersuchungsobjekt in x-, y- und z-Richtung gegenüber einem homogenen B0-Magnetfeld moduliert und die hochfrequenten Signale der Protonen über Antennenspulen detektiert werden. Durch die Modulation in z-Richtung lassen sich den Protonen einzelner Schichten eines Untersuchungsobjektes spezifische HF-Impulse zuteilen, welche für die Schichtenselektion verwendet werden können. Die Modulationen in x-Richtung und y-Richtung prägen den Protonen einen zusätzlichen Frequenzgradienten sowie einen Phasengradienten auf, damit die empfangenen Signale später einem Volumenelement im Untersuchungsobjekt zugeordnet werden können. Bei dreidimensionalen Messungen kann die Modulation in z-Richtung auch als zusätzliche, zweite Phasenkodierrichtung verwendet werden. Typischerweise wird das Summensignal der horizontalen Ortsfrequenzen anschließend in eine horizontale Zeile einer Matrix eingetragen, während das Summensignal der vertikalen Ortsfrequenzen in eine vertikale Spalte der Matrix eingetragen wird. Diese Matrix, auch k-Raum genannt, stellt den Datensatz der ersten Übersichtsmessung dar.

Die zweite Übersichtsmessung umfasst ein funktionelles Bildgebungsverfahren, wie z. B. PET oder SPECT. Das Ergebnis der zweiten Übersichtsmessung kann als Datenliste oder Matrix vorliegen, in welcher die über einen Detektor registrierten Ereignisse in festgelegten Zeitschritten aufsummiert und abgespeichert werden. Solche Ereignisse können z. B. die Aussendung eines Photons durch eine radioaktive Substanz darstellen oder eine Annihilierung eines Positrons mit einem Elektron, bei der zwei Photonen mit einer Energie von 511 keV in 180° versetzte Richtungen entsandt werden.

Die Aufnahme der ersten Übersichtsmessung überschneidet sich dabei zeitlich mit der zweiten Übersichtsmessung. Dies kann bedeuten, dass die erste Übersichtsmessung einen ersten Startzeitpunkt und einen ersten Endzeitpunkt aufweist und die zweite Übersichtsmessung einen zweiten Startzeitpunkt und einen zweiten Endzeitpunkt aufweist, wobei sich der Zeitraum der ersten Übersichtsmessung, welcher sich zwischen dem ersten Startzeitpunkt und dem ersten Endzeitpunkt erstreckt, mit dem Zeitraum der zweiten Übersichtsmessung, welcher sich zwischen dem zweiten Startzeitpunkt und dem zweiten Endzeitpunkt erstreckt, zumindest teilweise zeitlich überschneiden kann. Ein Betrag einer Differenz zwischen dem ersten Startzeitpunkt und dem zweiten Startzeitpunkt beträgt vorzugsweise maximal 30 Sekunden. Noch vorteilhafter ist ein Betrag von maximal 10 Sekunden. Besonders wünschenswert ist es, wenn der erste Startzeitpunkt mit dem zweiten Startzeitpunkt übereinstimmt. Eine zeitliche Überschneidung kann aber auch bedeuten, dass die Aufnahme der ersten Übersichtsmessung zumindest für einen festgelegten Zeitbereich zeitgleich zur Aufnahme der zweiten Übersichtsmessung erfolgt. Dieser festgelegte Zeitbereich ist vorteilhafterweise größer als 50 % der Messdauer der ersten Übersichtsmessung. Noch vorteilhafter ist es, wenn der festgelegte Zeitbereich mindesten 75 % der Messdauer der ersten Übersichtsmessung entspricht. Besonders wünschenswert ist eine Übereinstimmung des festgelegten Zeitbereichs mit der Messdauer der ersten Übersichtsmessung.

Dass die Aufnahme der ersten Übersichtsmessung sich zeitlich mit der Aufnahme der zweiten Übersichtsmessung überschneidet, kann weiterhin bedeuten, dass zumindest ein Zeitintervall vorliegt, während welchem sowohl Messdaten der ersten Übersichtsmessung als auch Messdaten der zweiten Übersichtsmessung akquiriert werden.

In einem nachfolgenden Schritt werden erste Übersichtsbilddaten basierend auf der ersten Übersichtsmessung und zweite Übersichtsbilddaten basierend auf der zweiten Übersichtsmessung erstellt. Hierfür werden die Übersichtsbilddaten auf Basis der Übersichtsmessungen insbesondere mit Hilfe von üblichen Verfahren zur Bildrekonstruktion rekonstruiert. Die Erstellung der ersten Übersichtsbilddaten erfolgt dabei typischerweise mittels einer Fourier-Transformation der Daten der ersten Übersichtsmessungen. Für die Erstellung der zweiten Übersichtsbilddaten werden vorzugsweise die orts- und zeitabhängigen Ereignisse ihrem jeweiligen Ursprung im Untersuchungsobjekt zugeordnet. Aus diesen Messdaten können anschließend durch Anwendung einer gefilterten Rückprojektion oder eines iterativen Rekonstruktionsverfahrens, wie z. B. Fourier-Rebinning und/oder OSEM ("ordered subset expectation maximization"), die zweiten Übersichtsbilddaten rekonstruiert werden. Die Erstellung der ersten Übersichtsbilddaten und der zweiten Übersichtsbilddaten wird vorzugsweise gestartet, sobald die Aufnahme der ersten Übersichtsmessung oder der zweiten Übersichtsmessung abgeschlossen ist und ein vollständiger Datensatz der ersten Übersichtsmessung bzw. der zweiten Übersichtsmessung vorliegt. Die erste Übersichtsmessung weist dabei insbesondere einen ersten Endzeitpunkt einer ersten Messdauer auf, während die zweite Übersichtsmessung insbesondere einen zweiten Endzeitpunkt der zweiten Messdauer aufweist. Liegt der erste Endzeitpunkt vor dem zweiten Endzeitpunkt kann mit der Erstellung der ersten Übersichtsbilddaten begonnen werden, sodass sich die Aufnahme der zweiten Übersichtsmessung mit der Erstellung der ersten Übersichtsbilddaten zeitlich überschneidet. Liegt dagegen der Endzeitpunkt der zweiten Übersichtsmessung vor dem Endzeitpunkt der ersten Übersichtsmessung, so kann mit der Erstellung der zweiten Übersichtsbilddaten begonnen werden, sodass sich die erste Übersichtsmessung mit der Erstellung der zweiten Übersichtsbilddaten zeitlich überschneidet. Daneben ist auch eine parallel zur ersten Übersichtsmessung ablaufende Rekonstruktion von ersten Übersichtsbilddaten, bzw. eine parallel zur zweiten Übersichtsmessung ablaufende Rekonstruktion von zweiten Übersichtsbilddaten vorstellbar. Vorteilhafterweise stimmen die Endzeitpunkte der ersten Übersichtsmessung und der zweiten Übersichtsmessung miteinander überein. Besonders wünschenswert ist es, wenn der Endzeitpunkt der ersten Übersichtsmessung vor dem Endzeitpunkt der zweiten Übersichtsmessung liegt.

In einem weiteren Schritt erfolgt eine Planung einer Messung des ersten Bildgebungsverfahrens und des zweiten Bildgebungsverfahrens auf Basis der ersten Übersichtsbilddaten und der zweiten Übersichtsbilddaten. Bei der Planung der Messung wird insbesondere ein Bildgebungsbereich und/oder ein Bildgebungsparameter des ersten Bildgebungsverfahrens und des zweiten Bildgebungsverfahrens festgelegt. Diese Festlegung erfolgt vorzugsweise anhand von Informationen über relevante physiologische und/oder pathologische Strukturen, welche aus den ersten Übersichtsbilddaten und den zweiten Übersichtsbilddaten abgeleitet werden. Die Planung der Messung erfolgt typischerweise auf Basis einer Analyse der Übersichtsbilddaten durch einen Benutzer des kombinierten Bildgebungsgeräts. Hierfür kann eine Anzeige der entsprechenden Bildinformationen auf einer Anzeigeeinheit des kombinierten Bildgebungsgeräts initiiert werden. Nach einer Analyse der Bildinformationen durch den Benutzer können die entsprechenden Bildgebungsbereiche und/oder Bildgebungsparameter mittels einer manuellen Eingabe festgelegt werden. Es ist aber auch die Anwendung entsprechender Algorithmen vorstellbar, welche physiologische und pathologische Strukturen in den ersten Übersichtsbilddaten und/oder den zweiten Übersichtsbilddaten identifizieren und dem Benutzer durch Anzeige auf einem Bildschirm kenntlich machen und/oder die Planung der Messung des ersten Bildgebungsverfahrens und des zweiten Bildgebungsverfahrens auf Basis der identifizierten Strukturen automatisch durchführen. Bei der Planung der Messung wird insbesondere der Bildgebungsbereich der Messung des ersten Bildgebungsverfahrens und/oder des zweiten Bildgebungsverfahrens festgelegt. Diese Festlegung kann z. B. eine Positionierung und/oder eine Anpassung einer Größe und/oder eine Ausrichtung des Bildgebungsbereiches umfassen. Alternativ oder zusätzlich kann bei der Planung der Messung auch zumindest ein Bildgebungsparameter der Messung festgelegt werden. Hiermit ist es insbesondere möglich, eine auf die physiologische oder pathologische Struktur abgestimmte Darstellung zu erhalten. Der zumindest eine Bildgebungsparameter kann zumindest einen Parameter oder eine beliebige Kombination von Parametern aus der folgenden, nicht als abschließende Aufzählung zu verstehenden Liste enthalten: eine Bildgebungssequenz auf Basis der T1- und T2-Relaxationszeiten, eine Schichtdicke der Aufnahme, eine Bewegungsgeschwindigkeit der Patientenlagerungsvorrichtung sowie eine Aufnahmedauer. Die im Rahmen der Planung festgelegten Bildgebungsbereiche und/oder Bildgebungsparameter werden vorzugsweise als Voreinstellungen abgespeichert.

In einem weiteren Verfahrensschritt erfolgt eine Durchführung der geplanten Messung des ersten Bildgebungsverfahrens und des zweiten Bildgebungsverfahrens, welche gegenüber einer Übersichtsmessung insbesondere einen höheren Informationsgehalt und/oder eine längere Messdauer aufweisen kann. Die geplante Messung umfasst typischerweise eine kombinierte Durchführung von Messungen mit dem ersten Bildgebungsverfahren und dem zweiten Bildgebungsverfahren. Vorzugsweise überschneidet sich die Messung mit dem ersten Bildgebungsverfahren wie oben beschrieben zeitlich mit der Messung mit dem zweiten Bildgebungsverfahren. Die während der Planung der Messung abgespeicherten Voreinstellungen können bei der Durchführung der Messung angewandt werden.

In einem letzten Verfahrensschritt werden Bilddaten des Untersuchungsobjektes auf Basis der Messung des ersten Bildgebungsverfahrens und des zweiten Bildgebungsverfahrens erstellt. Die Bilddaten werden insbesondere bereitgestellt. Das Bereitstellen der Bilddaten kann umfassen, dass die Bilddaten einem Benutzer auf einer Anzeigeeinheit angezeigt werden. Alternativ oder zusätzlich kann das Bereitstellen der Bilddaten umfassen, dass die Bilddaten in einer Datenbank abgespeichert werden. Alternativ oder zusätzlich kann das Bereitstellen der Bilddaten umfassen, dass die Bilddaten zunächst von einem Bildverarbeitungsalgorithmus weiterverarbeitet werden und anschließend angezeigt und/oder abgespeichert werden.

Durch die Berücksichtigung der ersten Übersichtsbilddaten und der zweiten Übersichtsbilddaten bei der Planung der Messung im Rahmen des erfindungsgemäßen Verfahrens weisen die Bilddaten insbesondere eine hohe Auflösung bezüglich der gewünschten physiologischen oder pathologischen Strukturen auf. Daneben kann eine auf die relevanten physiologischen und pathologischen Strukturen abgestimmte Positionierung der Bilddaten des ersten Bildgebungsverfahrens und des zweiten Bildgebungsverfahrens erreicht werden.

Das erfindungsgemäße Verfahren kann die Untersuchungsdauer für die Bildgebung mit einem kombinierten Bildgebungsgerät auf vorteilhafte Weise reduzieren. Auf Grundlage einer ersten Übersichtsmessung und den daraus erstellten ersten Übersichtsbilddaten sowie einer zweiten Übersichtsmessung und den daraus erstellten zweiten Übersichtsbilddaten können vorab Informationen über ein Untersuchungsobjekt akquiriert werden, welche insbesondere zu einer verbesserten Planung einer anschließenden Messung herangezogen werden. Der zeitliche Ablauf der Untersuchung kann somit besonders effizient gestaltet werden. Die Notwendigkeit der Erfassung weiterer Übersichtsbilddaten zur Anpassung der Bildgebungsparameter wird somit insbesondere vermieden.

Weitere vorteilhafte Ausgestaltungen und Weiterbildungen der Erfindung ergeben sich aus den abhängigen Ansprüchen sowie der nachfolgenden Beschreibung, wobei die unabhängigen Ansprüche einer Anspruchskategorie auch analog zu den abhängigen Ansprüchen einer anderen Anspruchskategorie weitergebildet sein können und die Merkmale verschiedener Ausführungsbeispiele zu neuen Ausführungsbeispielen kombiniert werden können.

In einer vorteilhaften Ausgestaltung des erfindungsgemäßen Verfahrens erfolgen die erste Übersichtsmessung und die zweite Übersichtsmessung bei einer kontinuierlichen Bewegung einer Patientenlagerungsvorrichtung des kombinierten Bildgebungsgeräts. Dies bedeutet, dass die kontinuierliche Bewegung der Patientenlagerungsvorrichtung zeitgleich zur Aufnahme von Messdaten mittels des kombinierten Bildgebungsgeräts erfolgen kann. Die kontinuierliche Bewegung der Patientenlagerungsvorrichtung erfolgt insbesondere in einer zeitlichen und örtlichen Abstimmung mit einer Durchführung des ersten Bildgebungsverfahrens und des zweiten Bildgebungsverfahrens, sodass zeitabhängige Übersichtsmessdaten des Untersuchungsobjektes aufgenommen werden. Darüber hinaus kann die kontinuierliche Bewegung der Patientenlagerungsvorrichtung auch abschnittsweise erfolgen, wobei die Patientenlagerungsvorrichtung zwischen den Abschnitten anhalten kann. Mit der Magnetresonanztomographie lässt sich pro Messintervall ein Querschnitt des Untersuchungsobjektes, d. h. ein quer zur Längsrichtung des Patientenaufnahmebereichs orientiertes Segment, erfassen. Ein solches Segment weist insbesondere eine definierte Schichtdicke auf, d. h. eine Abmessung in Längsrichtung des Patientenaufnahmebereichs, welche die Auflösung des Untersuchungsobjektes in dieser Richtung definiert. Die Bewegung der Patientenlagerungsvorrichtung und die Bildgebungssequenz der Magnetresonanztomographie werden dabei vorzugsweise aufeinander abgestimmt, sodass das Untersuchungsobjekt in einem Messintervall höchstens um den Betrag der Schichtdicke in der Längsrichtung bewegt wird. Durch dieses Vorgehen können erste Übersichtsmessungen des Untersuchungsobjektes aufgenommen werden, welche orts- und zeitabhängige Informationen des Untersuchungsobjektes enthalten. Ebenso können mit der zweiten Übersichtsmessung während der Bewegung der Patientenlagerungsvorrichtung orts- und zeitabhängige Übersichtsmessdaten aufgenommen werden, sofern das Bildgebungsverfahren, wie z. B. bei der Positronen-Emissionstomographie und der Einzel-Photonen-Emissionscomputertomographie, eine Ortskodierung der gemessenen Signale eines Untersuchungsobjekts aufweist. Durch die kontinuierliche Bewegung der Patientenlagerungsvorrichtung können Messdaten von Segmenten mit definierter Schichtdicke aufgenommen werden, welche das Untersuchungsobjekt abschnittsweise oder vollständig dreidimensional auflösen. Es ist vorstellbar, dass die Patientenlagerungsvorrichtung dabei einmal oder auch mehrfach durch den Patientenaufnahmebereich bewegt wird. Die Bewegung der Patientenlagerungsvorrichtung kann entweder in die Richtung der Öffnung des Patientenaufnahmebereichs oder in die entgegengesetzte Richtung weisen. Insbesondere kann sich die Bewegungsrichtung der Patientenlagerungsvorrichtung während der Aufnahme der Messdaten auch verändern. Wird die Patientenlagerungsvorrichtung während der Messdatenaufnahme mehrfach durch den Patientenaufnahmebereich bewegt, so lassen die jeweiligen Messdaten den gegenüber der Bewegung der Patientenlagerungsvorrichtung invarianten Ursprungsorten im Untersuchungsobjekt zuordnen. Durch das erfindungsgemäße Verfahren können dreidimensionale Übersichtsmessungen des Untersuchungsobjektes in geringer Zeit aufgenommen werden.

Gemäß einer Ausführungsform umfasst das zweite Bildgebungsverfahren eine Positronen-Emissionstomographie, bei welcher eine Anzahl von Annihilierungsereignissen unter kontinuierlicher Bewegung der Patientenlagerungsvorrichtung mittels eines Detektors zeitabhängig registriert und als zeitabhängiger Wert abgespeichert werden. Ein zeitabhängiger Wert stellt insbesondere ein registriertes Annihilierungsereignis dar, welches zusammen mit einem Zeitstempel abgespeichert wird. Dieser Zeitstempel kann z. B. den Zeitpunkt der Detektion des jeweiligen Annihilierungsereignisses enthalten. Die Dauer eines kleinsten Messintervalls, in welchem eine Anzahl von Annihilierungsereignissen registriert und aufsummiert wird, kann unabhängig vom Zeitschritt der ersten Übersichtsmessung sein. Das kleinste Messintervall des zweiten Bildgebungsverfahrens ist vorzugsweise zumindest mit dem Messintervall des ersten Bildgebungsverfahren abgestimmt. Besonders wünschenswert ist es, wenn die Messdauer zur vollständigen Erfassung eines quer zur Längsrichtung des Patientenaufnahmebereichs orientierten Segments mit definierter Schichtdicke bei dem ersten Bildgebungsverfahren und dem zweiten Bildgebungsverfahren übereinstimmen. Die Bewegungsgeschwindigkeit der Patientenlagerungsvorrichtung ist dabei vorzugsweise so auf beide Bildgebungsverfahren abgestimmt, dass eine ausreichende Messdatengrundlage erhalten wird, welche für die Rekonstruktion von dreidimensionalen, Übersichtsbilddaten eingesetzt werden kann. Durch diese Ausgestaltung des erfindungsgemäßen Verfahrens lassen sich die Vorteile der funktionellen Bildgebung der Positronen-Emissionstomographie mit den Vorteilen der strukturellen Bildgebung der Magnetresonanztomographie kombinieren. Die erhaltenen funktionellen und strukturellen Informationen über das Untersuchungsobjekt können auf vorteilhafte Weise zur zeiteffizienten Planung der Messung herangezogen werden.

In einer weiteren Ausgestaltungsform des erfindungsgemäßen Verfahrens umfasst die zweite Übersichtsmessung ein Zuordnen der registrierten Annihilierungsereignisse einem gegenüber der kontinuierlichen Bewegung der Patientenlagerungsvorrichtung invarianten Ursprung im Untersuchungsobjekt, wobei die kontinuierliche Bewegung der Patientenlagerungsvorrichtung berücksichtigt wird. Dabei können die in einem Zeitschritt registrierten Annihilierungsereignisse einem jeweils in dem Zeitschritt aufgenommenen, räumlichen Segment des Untersuchungsobjektes zugeordnet werden. Die Schichtdicke des räumlichen Segments hängt dabei insbesondere von einer Dauer des Zeitschritts sowie von einer Bewegungsgeschwindigkeit der Patientenlagerungsvorrichtung ab. Das Verfahren umfasst außerdem ein Abspeichern der zugeordneten, registrierten Annihilierungsereignisse als zweite Übersichtsmessung. Die räumlich zugeordneten, zeitabhängigen Annihilierungsereignisse können anschließend für eine Rekonstruktion der zweiten Übersichtsbilddaten herangezogen werden. Die Aufnahme der ersten Übersichtsmessung und der zweiten Übersichtsmessung stellt aufgrund der kontinuierlichen Bewegung der Patientenlagerungsvorrichtung insbesondere zeitlich korrelierte, dreidimensionale Messdaten der beiden Bildgebungsverfahren bereit. Diese dreidimensionalen Messdaten können auf vorteilhafte Weise zur Planung der Messung herangezogen werden können.

Da Photonen bei einer Positronen-Emissionstomographie-Messung typischerweise verschiedene Gewebestrukturen im Untersuchungsobjekt passieren, bevor sie auf den Detektor treffen, kann eine stärkere Abschwächung der Photonen aus dem Kern des Untersuchungsobjektes im Vergleich zu einem Punkt in unmittelbarer Nähe zur Oberfläche des Untersuchungsobjektes stattfinden. Diese Abschwächung hängt insbesondere mit dem Gewebe zusammen, welches die Photonen auf dem Weg zum Detektor passieren. Während Luft in den Lungen des Untersuchungsobjektes typischerweise nur eine geringe Abschwächung der Photonen verursacht, kann durch Weichgewebe oder feste Strukturen, wie z. B. Nierengewebe oder Knochen, ein höherer Anteil der Strahlung absorbiert werden. Dies führt insbesondere dazu, dass die Anzahl der registrierten Annihilierungsereignisse aus dem Kern des Untersuchungsobjektes geringer ist als aus den Randbereichen. Dies lässt sich als inhärenter Intensitätsunterschied zwischen Kern- und Randbereichen interpretieren, welcher mit Hilfe einer sogenannten Schwächungskarte kompensiert werden kann. Gemäß einer Ausführungsform wird daher auf Basis der ersten Übersichtsbilddaten eine Schwächungskarte des Untersuchungsobjektes erstellt, anhand derer eine Schwächungskorrektur der zweiten Übersichtsbilddaten erfolgt. Die Schwächungskarte ("µ-map") wird bevorzugt anhand von maximal vier Gewebearten erstellt. Vorteilhafterweise wird die Schwächungskarte anhand von mindestens zwei Gewebearten erstellt. Besonders wünschenswert ist eine Erstellung der Schwächungskarte anhand von drei Gewebearten. Beispielsweise wird die Schwächungskarte anhand von Luft, Lungengewebe und Weichgewebe erstellt. Insbesondere wird die Schwächungskarte anhand von Gewebearten erstellt, welche gut mittels Magnetresonanztomographie unterschieden werden können. Beispiele für Gewebearten, welche gut mittels Magnetresonanztomographie unterschieden werden können, stellen insbesondere das luftgefüllte Lungengewebe oder sonstige Weichgewebe im Untersuchungsobjekt dar. Die Unterscheidung der Gewebearten kann z. B. auf Basis der Graustufen und/oder Graubereichen der Bildpunkte in den ersten Übersichtsbilddaten erfolgen. Diesen Graustufen und/oder Graubereichen können Gewebetypen mit festgelegten Schwächungsfaktoren zugeordnet werden, mit denen die Schwächung der Photonen im Untersuchungsobjekt kompensiert wird. Peripheriekomponenten des kombinierten Bildgebungsgeräts, welche während der Untersuchung zwischen Detektor und Untersuchungsobjekt positioniert sind, wie z. B. die Patientenlagerungsvorrichtung oder flexible Lokalspulen, lassen sich ebenfalls bei der Schwächungskorrektur berücksichtigen. Hierfür kann z. B. eine einmalige Messung der auftretenden Schwächung als Kalibrierung durchgeführt werden, da sich die Schwächungswerte über die Lebensdauer dieser Komponenten typischerweise nur geringfügig ändern. Um eine automatische Kompensation solcher Peripheriekomponenten zu erreichen, können diese mit Magnetresonanz-aktiven Markern (MR-Phantome) versehen und bei der Erstellung der Schwächungskarte berücksichtigt werden. Die Planung der Messung erfolgt auf Basis der schwächungskorrigierten zweiten Übersichtsbilddaten. Dies ist vorteilhaft, da die Aussagekraft der zweiten Übersichtsbilddaten durch eine Schwächungskorrektur erheblich verbessert werden kann. Ist die Qualität der zweiten, nichtschwächungskorrigierten Übersichtsbilddaten nicht ausreichend für eine Planung der Messung, können die schwächungskorrigierten zweiten Übersichtsbilddaten herangezogen werden, statt eine weitere zweite Übersichtsmessung aufzunehmen und daraus erneut zweite Übersichtsbilddaten zu rekonstruieren. Dadurch kann die Untersuchungsdauer vorteilhaft reduziert werden.

In einer bevorzugten Vorgehensweise wird auf Basis der bei kontinuierlicher Bewegung der Patientenlagerungsvorrichtung aufgenommenen ersten Übersichtsmessung eine Schwächungskarte erstellt, wobei die Schwächungskarte zur Schwächungskorrektur der zweiten Übersichtsbilddaten, welche ihrem gegenüber der kontinuierlichen Bewegung der Patientenlagerungsvorrichtung invarianten Ursprung im Untersuchungsobjekt zugeordnete Annihilierungsereignisse enthalten, erfolgt. Da die ersten Übersichtsbilddaten aufgrund der kontinuierlichen Bewegung der Patientenlagerungsvorrichtung dreidimensionale Informationen über das Untersuchungsobjekt enthalten, kann anhand der daraus abgeleiteten Schwächungskarte für jedes Voxel der dreidimensionalen Abbildung des Untersuchungsobjekts ein Schwächungskorrekturwert abgeleitet werden. Je nach Untersuchungsablauf können bei Aufnahme der ersten Übersichtsmessung ein Abschnitt, mehrere Abschnitte oder das gesamte Untersuchungsobjekt bei kontinuierlicher Bewegung der Patientenlagerungsvorrichtung untersucht werden. Für alle dieser aufgenommenen Abschnitte liegen dann dreidimensionale Informationen vor, welche für die Erstellung einer Schwächungskarte verwendet werden können. Mit Hilfe dieser Schwächungskarte lässt sich auf vorteilhafte Weise eine Schwächungskorrektur der Ausschnitte der zweiten Übersichtsbilddaten durchführen. Die ersten Übersichtsbilddaten, aus denen die Schwächungskarte abgeleitet wird, können mit einem geringen Zeitaufwand erstellt werden. Dadurch kann der Zeitaufwand für die gesamte Untersuchungsdauer auf vorteilhafte Weise minimiert werden.

In einer weiteren möglichen Ausführungsform wird nach der Aufnahme der ersten Übersichtsmessung eine dritte Übersichtsmessung mit einem gegenüber der ersten Übersichtsmessung erweitertem Bildgebungsbereich mittels des ersten Bildgebungsverfahrens durchgeführt, wobei anhand der ersten Übersichtsmessung und der dritten Übersichtsmessung erweiterte erste Übersichtsbilddaten erstellt werden. Ein üblicher Bildgebungsbereich für einen Magnetresonanztomographen umfasst beispielsweise eine Abmessung von etwa 50 bis 60 cm quer zur Längsrichtung des Patientenaufnahmebereichs des kombinierten Bildgebungsgeräts. Um Bilder des Untersuchungsobjektes in der Längserstreckung des Patientenaufnahmebereichs aufzunehmen, werden typischerweise konsekutive Messungen bei kontinuierlicher oder diskontinuierlicher Bewegung der Patientenlagerungsvorrichtung durchgeführt. Für die Erweiterung des Bildgebungsbereiches in Querrichtung zum Patientenaufnahmebereich müssen dagegen separate Aufnahmen mit einem erweiterten Bildgebungsbereich durchgeführt werden, die sich der ersten Übersichtsmessung als dritte Übersichtsmessung anschließen können. Die Aufnahme einer dritten Übersichtsmessung kann sich zeitlich mit der Erstellung der zweiten Übersichtsbilddaten überschneiden. Dies bedeutet, dass die Aufnahme der dritten Übersichtsmessung einen Startzeitpunkt besitzt, welcher sich an den Endzeitpunkt einer letzten Übersichtsmessung anschließen kann. Eine letzte Übersichtsmessung stellt beispielsweise eine erste Übersichtsmessung oder eine zweite Übersichtsmessung dar, deren durch den Startzeitpunkt und Endzeitpunkt definierte Messdauer größer ist, als die der jeweils anderen. Liegt der Endzeitpunkt der ersten Übersichtsmessung nach dem Endzeitpunkt der zweiten Übersichtsmessung, so kann sich die dritte Übersichtsmessung an die erste Übersichtsmessung anschließen. Liegt dagegen der Endzeitpunkt der zweiten Übersichtsmessung nach dem Endzeitpunkt der ersten Übersichtsmessung, so kann sich die dritte Übersichtsmessung an die zweite Übersichtsmessung anschließen. Dies bedeutet, dass der Startzeitpunkt der dritten Übersichtsmessung vorzugsweise maximal 30 Sekunden nach dem Endzeitpunkt der letzten Übersichtsmessung liegt. Vorteilhafterweise sollte der Startzeitpunkt der dritten Übersichtsmessung maximal 20 Sekunden nach dem Endzeitpunkt der letzten Übersichtsmessung liegen. Noch vorteilhafter ist eine Differenz von maximal 10 Sekunden zwischen Starzeitpunkt der dritten Übersichtsmessung und Endzeitpunkt der letzten Übersichtsmessung. Besonders wünschenswert ist es, wenn sich der Startzeitpunkt der dritten Übersichtsmessung ohne Verzögerung an den Endzeitpunkt der letzten Übersichtsmessung anschließt. Die Aufnahme von dritten Übersichtsmessungen ist insbesondere dann erwägenswert, wenn die Abmessung des Untersuchungsobjekts den Bildgebungsbereich quer zur Längsrichtung des Patientenaufnahmebereichs überschreitet und/oder diagnostisch relevante Informationen in den außerhalb des Bildgebungsbereichs lokalisierten Teilen des Untersuchungsobjektes zu erwarten sind. Beispiele hierfür sind außerhalb des Bildgebungsbereichs positionierte Extremitäten eines Patienten, wie z. B. Schultern oder Ellenbogen. Die außerhalb des Bildgebungsbereichs positionierten Extremitäten sind insbesondere dann relevant, wenn sie pathologische Strukturen aufweisen, welche bei der Untersuchung zu berücksichtigen sind. Auf Basis der erweiterten ersten Übersichtsbilddaten kann die Analyse der Übersichtsbilddaten durch den Benutzer des kombinierten Bildgebungsgeräts unterstützt werden. Die Planung der Messung lässt sich auf diese Weise entsprechend verbessern.

Eine weitere Ausführungsform sieht vor, dass die Schwächungskarte des Untersuchungsobjektes anhand der erweiterten ersten Übersichtsbilddaten erstellt wird. Auf Basis dieser Schwächungskarte wird eine Schwächungskorrektur der zweiten Übersichtsbilddaten durchgeführt, wobei die Planung der Messung anhand der schwächungskorrigierten zweiten Übersichtsbilddaten erfolgt. Die Schwächungskarte wird vorzugsweise nach dem oben beschriebenen Verfahren erstellt, berücksichtigt aber auch die außerhalb des üblichen Bildgebungsbereiches lokalisierten Teile des Untersuchungsobjektes. Dadurch lässt sich die Schwächung von Signalen in den außerhalb des üblichen Bildgebungsbereichs liegenden Teilen des Untersuchungsobjektes kompensieren. Dies kann die Aussagekraft der schwächungskorrigierten zweiten Übersichtsbilddaten signifikant erhöhen.

Auf Basis der ersten Übersichtsbilddaten, der erweiterten ersten Übersichtsbilddaten und der zweiten Übersichtsbilddaten kann eine Anzeige der entsprechenden Bildinformationen auf einer entsprechenden Anzeigeeinheit für den Benutzer des kombinierten Bildgebungsgeräts initiiert werden. Die Bilddaten der unterschiedlichen Bildgebungsverfahren können dabei separat voneinander dargestellt werden, um die entsprechenden Bildinformationen übersichtlich zu präsentieren. In einigen Fällen ist es jedoch erwägenswert die Bildinformationen der beiden Bildgebungsverfahren in einer Darstellung zu kombinieren, z. B. um den Abstand einer pathologischen Struktur, die sich aus der funktionellen Bildgebung ableitet, zu einer benachbarten physiologischen Struktur, welche sich aus einer strukturellen Bildgebung ableitet, festzustellen. In einer weiteren Ausführungsform werden daher auf Basis der ersten Übersichtsbilddaten oder den erweiterten ersten Übersichtsbilddaten und den zweiten Übersichtsbilddaten kombinierte Übersichtbilddaten erstellt. Diese enthalten die Informationen aus den Übersichtsbilddaten des ersten und des zweiten Bildgebungsverfahrens, welche im Rahmen einer zwei- oder dreidimensionalen Abbildung, z. B. eines Abschnitts, mehrerer Abschnitte oder des gesamten Untersuchungsobjektes, auf der Anzeigeeinheit des kombinierten Bildgebungsgeräts visualisiert werden können. Für eine bessere Unterscheidung und eine übersichtliche Darstellung können den Bildpunkten der beiden Bildgebungsverfahren verschiedene Intensitäten, z. B. unterschiedliche Graustufenbereiche, Farbbereiche oder Transparenzen, zugeordnet werden. Die kombinierten Übersichtsbilddaten werden zur Planung der Messung herangezogen. Dadurch kann dem Benutzer des kombinierten Bildgebungsgeräts die Analyse der Übersichtsbilddaten erleichtert werden. Dies verbessert auf vorteilhafte Weise die Planung der Messung.

Gemäß einer Ausführungsform weisen die zweiten Übersichtsbilddaten gegenüber den ersten Übersichtsbilddaten Informationen über Regionen mit erhöhter funktioneller Aktivität im Untersuchungsobjekt auf. Um den Bildgebungsbereich und/oder die Bildgebungsparameter der geplanten Messung auf die entsprechenden Regionen mit erhöhter funktioneller Aktivität anzupassen, werden daher insbesondere die zweiten Übersichtsbilddaten auf pathologische Strukturen hin untersucht. Diese Untersuchung kann z. B. durch Bildverarbeitungsalgorithmen erfolgen, welche pathologische Strukturen im Untersuchungsobjekt automatisch identifizieren und dem Benutzer des kombinierten Bildgebungsgeräts anzeigen. Solche Bildverarbeitungsalgorithmen können beispielsweise auf künstlichen neuronalen Netzen oder Expertensystemen beruhen. Die Informationen des Bildverarbeitungsalgorithmus können auch dazu eingesetzt werden, eine Optimierung des Bildgebungsbereiches und/oder der Bildgebungsparameter durchzuführen. Es ist ebenso vorstellbar, dass der Benutzer selbst auf Basis einer Analyse der zweiten Übersichtsbilddaten eine Festlegung des Bildgebungsbereiches und/oder der Bildgebungsparameter vornimmt. Die Planung der Messung erfolgt auf Basis der identifizierten Regionen mit erhöhter funktioneller Aktivität. Dies kann z. B. bedeuten, dass ein Bildgebungsbereich eines Bildgebungsverfahrens mit einer Region erhöhter Aktivität abgestimmt ist. Eine Abstimmung des Bildgebungsbereiches mit der Region erhöhter Aktivität kann insbesondere eine Anpassung einer Größe, einer Form oder einer Position des Bildgebungsbereiches an die betreffende Region umfassen. Weitere, nicht als abschließende Aufzählung zu verstehende, Möglichkeiten der Einflussnahme auf die Planung der Messung stellen z. B. die Anpassung von Kontrasten über die T1- und T2-Relaxationszeiten oder der Aufnahmedauer des zweiten Bildgebungsverfahrens dar. Auf diese Weise lassen sich funktionell aktive, pathologische Strukturen zeiteffizient auf Basis der zweiten Übersichtsbilddaten darstellen und bei der Planung der Messung berücksichtigen.

In einer weiteren Vorgehensweise weisen die kombinierten Übersichtsbilddaten gegenüber den ersten Übersichtsbilddaten und den zweiten Übersichtsbilddaten erweiterte Informationen zu pathologischen Strukturen und ihren Positionen im Untersuchungsobjekt auf, wobei die kombinierten Übersichtsbilddaten auf pathologische Strukturen hin untersucht werden und die Planung der Messung auf Basis der identifizierten pathologischen Strukturen erfolgt. Die kombinierten Übersichtsbilddaten enthalten sowohl funktionelle als auch strukturelle Informationen über das Untersuchungsobjekt und können damit die Lokalisierung sowie die Interpretation diagnostisch relevanter Strukturen erleichtern. Dem Benutzer des kombinierten Bildgebungsgeräts werden auf Basis der kombinierten Übersichtsbilddaten zumindest zweidimensionale, vorzugsweise dreidimensionale Abbildungen des Untersuchungsobjektes zur Verfügung gestellt, welche durch eine grafische Aufbereitung im Rahmen der üblichen Schnittebenen, z. B. der axialen, sagittalen oder koronalen Schnittebene, vom Benutzer angepasst werden können. Es ist vorstellbar, dass die Regionen mit erhöhter funktioneller Aktivität im Untersuchungsobjekt auf Basis der Informationen der kombinierten Übersichtsbilddaten von entsprechenden Algorithmen identifiziert und bei der anschließenden Messung im Bildgebungsbereich zentriert gemessen werden. Dadurch kann verhindert werden, dass relevante Strukturen außerhalb des Bildgebungsbereiches liegen. Es ist weiterhin vorstellbar, dass bestimmte Regionen ohne funktionelle Aktivität bei der Messung ausgespart werden, um die Messung zu beschleunigen. Identifizierte Regionen mit funktioneller Aktivität können anschließend automatisch in der Visualisierung der kombinierten Übersichtsbilddaten hervorgehoben und/oder zur Optimierung des Bildgebungsbereichs sowie der Bildgebungsparameter bei der Planung der Messung herangezogen werden. Dadurch lassen sich die Aussagekraft der erhaltenen Informationen erhöhen und die Analyse der kombinierten Übersichtsbilddaten durch den Benutzer des kombinierten Bildgebungsgeräts auf vorteilhafte Weise erleichtern. Der Benutzer kann auf Basis der kombinierten Übersichtsbilddaten eine verbesserte Planung der Messung durchführen und eine wiederholte Aufnahme von ersten und zweiten Übersichtsmessungen vermeiden.

Das erfindungsgemäße kombinierte Bildgebungsgerät umfasst eine Messeinheit und ist zur Durchführung des Verfahrens nach einem der vorangegangenen Ansprüche ausgebildet.

Die Messeinheit des kombinierten Bildgebungsgeräts ist dazu ausgebildet, eine erste Übersichtsmessung mittels eines ersten Bildgebungsverfahrens sowie eine zweite Übersichtsmessung mittels eines zweiten Bildgebungsverfahrens durchzuführen. Die Durchführung der ersten Übersichtsmessung überschneidet sich analog zum erfindungsgemäßen Verfahren zeitlich mit der Durchführung der zweiten Übersichtsmessung.

Eine mögliche Weiterbildung der Messeinheit sieht vor eine dritte Übersichtsmessung mittels eines ersten Bildgebungsverfahrens nach den oben beschriebenen Verfahrensschritten durchzuführen, wobei die dritte Übersichtsmessung nach dem Endzeitpunkt der jeweiligen Übersichtsmessung mit der größeren Messdauer erfolgt.

Die Messeinheit des erfindungsgemäßen Bildgebungsgerätes weist eine Recheneinheit sowie eine Planungseinheit auf. Die Recheneinheit der erfindungsgemäßen Messeinheit ist dazu ausgebildet, erste Übersichtsbilddaten auf Basis der ersten Übersichtsmessung und zweite Übersichtsbilddaten auf Basis der zweiten Übersichtsmessung zu erstellen.

In einer möglichen Weiterbildung ist die Recheneinheit dazu ausgebildet, bei Vorliegen von weiteren Übersichtsmessungen, wie z. B. einer dritten Übersichtsmessung, auf Basis der Informationen einer ersten Übersichtsmessung und einer dritten Übersichtsmessung eines ersten Bildgebungsverfahrens, erweiterte erste Übersichtsbilddaten zu erstellen.

In einer weiteren möglichen Ausführungsform ist die erfindungsgemäße Recheneinheit dazu ausgebildet, erste Übersichtsbilddaten und zweite Übersichtsbilddaten oder erweiterte erste Übersichtsbilddaten und zweite Übersichtsbilddaten, zu kombinierten Übersichtsbilddaten zusammenzuführen.

Die Planungseinheit der erfindungsgemäßen Messeinheit ist dazu ausgebildet, ein Planen einer Messung mittels eines ersten Bildgebungsverfahrens des kombinierten Bildgebungsgeräts durchzuführen, wobei das Planen der Messung ein Festlegen von ersten Bildgebungsbereichen und ersten Bildgebungsparametern für die geplante Messung umfassen kann, wobei das Festlegen von ersten Bildgebungsbereichen und ersten Bildgebungsparametern insbesondere auf Basis von zweiten Übersichtsbilddaten erfolgt.

Weiterhin ist die Planungseinheit dazu ausgebildet, ein Planen einer Messung mittels eines zweiten Bildgebungsverfahrens des kombinierten Bildgebungsgeräts durchzuführen, wobei das Planen der Messung ein Festlegen von zweiten Bildgebungsbereichen und zweiten Bildgebungsparametern für die zweite Messung umfassen kann, wobei das Festlegen von zweiten Bildgebungsbereichen und zweiten Bildgebungsparametern insbesondere auf Basis von zweiten Übersichtsbilddaten erfolgt.

Weiterhin ist die Messeinheit, insbesondere mit einem kombinierten Bildgebungsgerät, dazu ausgebildet, die geplante Messung des ersten Bildgebungsverfahrens des zweiten Bildgebungsverfahrens durchzuführen.

Ausführungsformen des erfindungsgemäßen kombinierten Bildgebungsgeräts sind analog zu den Ausführungsformen des erfindungsgemäßen Verfahrens ausgebildet. Die erfindungsgemäße Messeinheit ermöglicht somit ein effizientes Ausnutzen der Untersuchungsdauer eines kombinierten Bildgebungsgeräts. Die Messeinheit kann weitere Steuerungskomponenten aufweisen, welche zum Ausführen eines erfindungsgemäßen Verfahrens nötig und/oder vorteilhaft sind. Auch kann die Messeinheit dazu ausgebildet sein, Steuerungssignale an ein kombiniertes Bildgebungsgerät zu senden und/oder Steuerungssignale zu empfangen und/oder zu verarbeiten, um ein erfindungsgemäßes Verfahren auszuführen. Die Recheneinheit der Messeinheit ist vorteilhafterweise dazu ausgebildet, um Prozesse innerhalb des kombinierten Bildgebungsgeräts aufeinander abzustimmen. Auf einer Speichereinheit der Messeinheit können Computerprogramme und weitere Software gespeichert sein, mittels derer ein Prozessor der Messeinheit einen Verfahrensablauf eines erfindungsgemäßen Verfahrens automatisch steuert und/oder ausführt.

Das erfindungsgemäße kombinierte Bildgebungsgerät weist eine Messeinheit auf. Damit ist das erfindungsgemäße kombinierte Bildgebungsgerät dazu ausgebildet, mit der Messeinheit ein erfindungsgemäßes Verfahren auszuführen. Die Messeinheit kann mit dem kombinierten Bildgebungsgerät verbunden, in diesem integriert oder separat von diesem installiert sein. Das erfindungsgemäße kombinierte Bildgebungsgerät ermöglicht somit ein optimales Ausnutzen der Untersuchungsdauer des kombinierten Bildgebungsgeräts.

Das erfindungsgemäße Computerprogrammprodukt ist direkt in einen Speicher einer programmierbaren Recheneinheit eines kombinierten Bildgebungsgeräts ladbar und weist Programmcode-Mittel auf, um ein erfindungsgemäßes Verfahren auszuführen, wenn das Computerprogrammprodukt in der Recheneinheit des kombinierten Bildgebungsgeräts ausgeführt wird. Dadurch kann das erfindungsgemäße Verfahren schnell, identisch wiederholbar und robust ausgeführt werden. Das Computerprogrammprodukt ist so konfiguriert, dass es mittels der Recheneinheit die erfindungsgemäßen Verfahrensschritte ausführen kann. Die Recheneinheit muss dabei jeweils die Voraussetzungen wie beispielsweise einen entsprechenden Arbeitsspeicher, eine entsprechende Grafikkarte oder eine entsprechende Logikeinheit aufweisen, so dass die jeweiligen Verfahrensschritte effizient ausgeführt werden können. Das Computerprogrammprodukt ist beispielsweise auf einem computerlesbaren Medium gespeichert oder auf einem Netzwerk, einem Server oder einer Cloud hinterlegt, von wo es in den Prozessor einer lokalen Recheneinheit geladen werden kann, der mit dem kombinierten Bildgebungsgerät direkt verbunden oder als Teil des kombinierten Bildgebungsgeräts ausgebildet sein kann. Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in einer Recheneinheit eines kombinierten Bildgebungsgeräts ein erfindungsgemäßes Verfahren durchführen. Beispiele für elektronisch lesbare Datenträger sind eine DVD, ein Magnetband oder einen USB-Stick, auf welchem elektronisch lesbare Steuerinformationen, insbesondere Software (vgl. oben), gespeichert ist. Wenn diese Steuerinformationen (Software) von dem Datenträger gelesen und in eine Steuerung und/oder Recheneinheit eines kombinierten Bildgebungsgeräts gespeichert werden, können alle erfindungsgemäßen Ausführungsformen der vorab beschriebenen Verfahren durchgeführt werden.

Die Vorteile der erfindungsgemäßen Messeinheit, des erfindungsgemäßen kombinierten Bildgebungsgeräts, und des erfindungsgemäßen Computerprogrammprodukts entsprechen im Wesentlichen den Vorteilen des erfindungsgemäßen Verfahrens, welche vorab im Detail ausgeführt sind. Hierbei erwähnte Merkmale, Vorteile oder alternative Ausführungsformen sind ebenso auf die anderen beanspruchten Gegenstände zu übertragen und umgekehrt. Mit anderen Worten können die gegenständlichen Ansprüche auch mit den Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Module, insbesondere durch Hardware-Module, ausgebildet.

Im Folgenden wird die Erfindung anhand der in den Figuren dargestellten Ausführungsbeispiele näher beschrieben und erläutert.

Es zeigen:
- Fig. 1: eine mögliche Ausführungsform eines kombinierten Bildgebungsgeräts zur Ausführung eines erfindungsgemäßen Verfahrens in einer schematischen Darstellung,
- Fig. 2: einen schematischen Ablaufplan gemäß einer Ausführungsform erfindungsgemäßen Verfahrens,
- Fig. 3: einen zeitlichen Ablaufplan gemäß einer Ausführungsform eines erfindungsgemäßen Verfahrens, bei welchem eine Schwächungskorrektur von zweiten Übersichtsbilddaten durchgeführt wird,
- Fig. 4: PET-Übersichtsbilddaten und MR-Übersichtsbilddaten, welche gemäß einer Ausführungsform des erfindungsgemäßen Verfahrens erstellt werden.

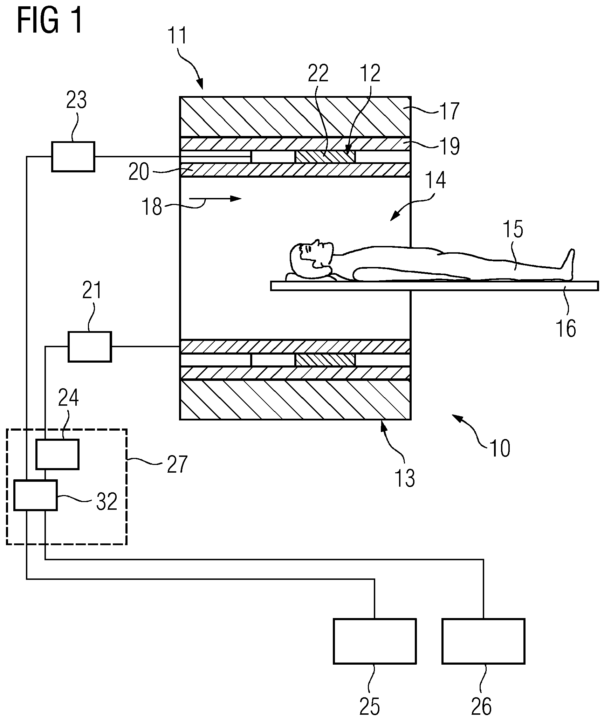

Fig. 1 zeigt ein kombiniertes Bildgebungsgerät 10 zur Ausführung eines erfindungsgemäßen Verfahrens in einer schematischen Darstellung. Das kombinierte Bildgebungsgerät 10 ist im gezeigten Fall ein Magnetresonanz-Positronen-Emissionstomographie-Gerät 10 (MR-PET-Gerät) und weist zwei Bildgebungsverfahren auf. Dabei ist das erste Bildgebungsverfahren die Magnetresonanztomographie und das zweite Bildgebungsverfahren die Positronen-Emissionstomographie.

Das zweite Bildgebungsverfahren kann alternativ eine Positronen-Emissionstomographie (PET), Einzelphotonenemissionstomographie (SPECT), Computertomographie, Ultraschall-Bildgebung, Röntgen-Bildgebung oder C-Bogen-Bildgebung umfassen. Es sind dabei beliebige Kombinationen aus dem ersten Bildgebungsverfahren und dem zweiten Bildgebungsverfahren denkbar.

Das MR-PET-Gerät umfasst eine Magnetresonanztomographie-Vorrichtung 11 (MRT-Vorrichtung 11) und eine Positronen-Emissionstomographie-Vorrichtung 12 (PET-Vorrichtung 12). Die MRT-Vorrichtung 11 stellt exemplarisch das erste Bildgebungsverfahren des MR-PET-Geräts 10 dar, während die PET-Vorrichtung 12 das zweite Bildgebungsverfahren des MR-PET-Geräts 10 darstellt. Das kombinierte Bildgebungsgerät 10 kann selbstverständlich auch eine andere Kombination von Bildgebungsverfahren enthalten. In Fig. 1 bis Fig. 4 wird das erfindungsgemäße Verfahren lediglich zur Veranschaulichung anhand eines konkreten MR-PET-Geräts 10 dargestellt.

Die MRT-Vorrichtung 11 umfasst eine Magneteinheit 13 und einen von der Magneteinheit 13 umgebenen Patientenaufnahmebereich 14 zu einer Aufnahme eines Untersuchungsobjekts 15, insbesondere eines Patienten 15, wobei der Patientenaufnahmebereich 14 in einer Umfangsrichtung von der Magneteinheit 13 zylinderförmig umgeben ist. Der Patient 15 kann mittels einer Patientenlagerungsvorrichtung 16 der MRT-Vorrichtung 11 in den Patientenaufnahmebereich 14 geschoben werden. Die Patientenlagerungsvorrichtung 16 ist hierzu bewegbar innerhalb des Patientenaufnahmebereichs 16 angeordnet.

Die Magneteinheit 13 umfasst einen Hauptmagneten 17, der im Betrieb der MRT-Vorrichtung 11 zu einer Erzeugung eines starken und insbesondere konstanten Hauptmagnetfelds 18 ausgelegt ist. Die Magneteinheit 13 weist weiterhin eine Gradientenspuleneinheit 19 zu einer Erzeugung von Magnetfeldgradienten auf, die für eine Ortskodierung während einer Bildgebung verwendet wird. Die Gradientenspuleneinheit 19 ist weiterhin zur Erzeugung von Gradientenfeldern ausgebildet. Zudem umfasst die Magneteinheit 13 eine Körperspule 20, die zu einer Anregung einer Polarisation vorgesehen ist, die sich in dem von dem Hauptmagneten 17 erzeugten Hauptmagnetfeld 18 einstellt. Die Körperspule 20 ist weiterhin zum Empfang von Magnetresonanz-Signalen vorgesehen. Die Körperspule 20 ist zum Empfang einer ersten und zweiten Signal-Frequenz ausgebildet. Die Körperspule 20 ist fest innerhalb der Magneteinheit integriert.

Zu einer Steuerung des Hauptmagneten 17 der Gradientenspuleneinheit 19 sowie einer Steuerung der Körperspule 20 weist das MR-PET-Gerät 10, insbesondere die MRT-Vorrichtung 11, eine Magnetresonanz-Steuereinheit 21 auf. Die Magnetresonanz-Steuereinheit 21 steuert zentral die MRT-Vorrichtung 11, wie beispielsweise das Durchführen einer vorbestimmten bildgebenden Gradientenechosequenz. Hierzu umfasst die Magnetresonanz-Steuereinheit 21 eine nicht näher dargestellte Gradientensteuereinheit und eine nicht näher dargestellte Hochfrequenzantennensteuereinheit. Zudem umfasst die Magnetresonanz-Steuereinheit 21 eine Auswerteeinheit zu einer Auswertung von Magnetresonanz-Bilddaten oder Magnetresonanz-Signalen.

Die dargestellte MRT-Vorrichtung 11 kann selbstverständlich weitere Komponenten umfassen, die MRT-Vorrichtungen 11 gewöhnlich aufweisen. Eine allgemeine Funktionsweise einer MRT-Vorrichtung 11 ist zudem dem Fachmann bekannt, so dass auf eine detaillierte Beschreibung der allgemeinen Komponenten verzichtet wird.

Die PET-Vorrichtung 12 umfasst mehrere Positronen-Emissions-Tomographie-Detektormodule 22 (PET-Detektormodule 22), die zu einer Ringform angeordnet sind und den Patientenaufnahmebereich 14 in der Umfangsrichtung umgeben. Die PET-Detektormodule 22 weisen jeweils mehrere, nicht näher dargestellte Positronen-Emissions-Tomographie-Detektorelemente (PET-Detektorelemente) auf, die zu einem PET-Detektorarray angeordnet sind, das ein Szintillationsdetektorarray mit Szintillationskristallen, beispielsweise LSO-Kristalle, umfasst. Des Weiteren umfassen die PET-Detektormodule 22 jeweils ein Photodiodenarray, beispielsweise Avalanche-Photodiodenarray oder APD-Photodiodenarray, die dem Szintillationsdetektorarray nachgeschaltet innerhalb der PET-Detektormodule 22 angeordnet sind. Mittels der PET-Detektormodule 22 werden Photonenpaare, die aus der Annihilation eines Positrons mit einem Elektron resultieren, erfasst. Das Positron wird hierbei von einem Radiopharmakon emittiert, wobei das Radiopharmakon über eine Injektion dem Patienten 15 verabreicht wird.

Zudem weisen die PET-Detektormodule 22 jeweils eine Detektorelektronik auf, die eine elektrische Verstärkerschaltung und weitere, nicht näher dargestellte Elektronikkomponenten umfasst. Zu einer Steuerung der Detektorelektronik und der PET-Detektormodule 22 weist das MR-PET-Gerät 10, insbesondere die PET-Vorrichtung 12, eine PET-Steuereinheit 23 auf. Die PET-Steuereinheit 23 steuert zentral die PET-Vorrichtung 12. Zudem umfasst die PET-Steuereinheit 23 eine Auswerteeinheit zu einer Auswertung von PET-Daten.

Die dargestellte PET-Vorrichtung 12 kann selbstverständlich weitere Komponenten umfassen, die PET-Vorrichtungen 12 gewöhnlich aufweisen. Eine allgemeine Funktionsweise einer PET-Vorrichtung 12 ist zudem dem Fachmann bekannt, so dass auf eine detaillierte Beschreibung der allgemeinen Komponenten verzichtet wird.

Das MR-PET-Gerät 10 weist eine Messeinheit 27 zur Durchführung einer Messung des MR-PET-Geräts 10 auf, wobei die Messeinheit 27 eine Planungseinheit 32 und eine Recheneinheit 24 umfasst. Das MR-PET-Gerät 10, insbesondere die Messeinheit 27, sind dabei dazu ausgebildet, ein erfindungsgemäßes Verfahren auszuführen.

Die Recheneinheit 24 der Messeinheit 27 kann eine Komponente der Messeinheit 27 und/oder eine zentrale Systemsteuereinheit des MR-PET-Geräts 10 sein. Die Recheneinheit 24 stimmt beispielsweise eine Erfassung und/oder eine Auswertung von MR-Bilddaten und von PET-Bilddaten aufeinander ab.

Steuerinformationen wie beispielsweise Bildgebungsparameter, sowie rekonstruierte Bilddaten können auf einer Anzeigeeinheit 25, beispielsweise auf zumindest einem Monitor, des MR-PET-Geräts 10 für einen Benutzer angezeigt werden. Zudem weist das MR-PET-Gerät 10 eine Eingabeeinheit 26 auf, mittels welcher Informationen und/oder Parameter während einer Untersuchung von einem Benutzer eingegeben werden können. Die Recheneinheit 24 kann die Magnetresonanz-Steuereinheit 21 und/oder die PET-Steuereinheit 23 umfassen.

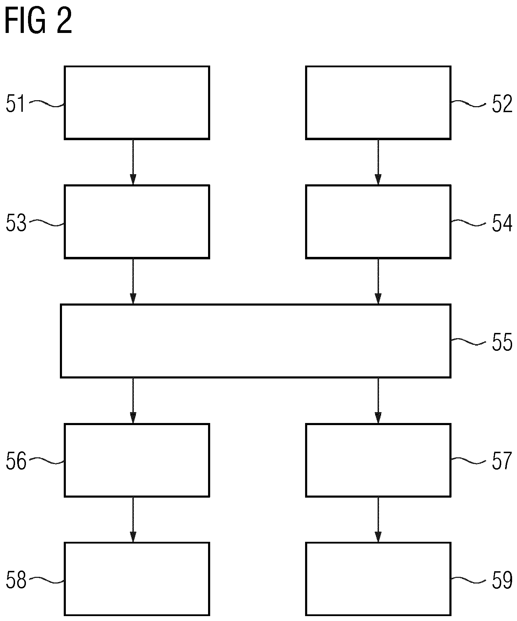

Fig. 2 zeigt ein Ablaufdiagramm einer möglichen Ausführungsform des erfindungsgemäßen Verfahrens. Im ersten Verfahrensschritt 51 wird eine MR-Übersichtsmessung mittels der MR-Vorrichtung 11 und der Messeinheit 27 des kombinierten Bildgebungsgeräts 10 aufgenommen. In einem weiteren Verfahrensschritt 52 wird zeitgleich zum Verfahrensschritt 51 eine PET-Übersichtsmessung mittels der PET-Vorrichtung 12 und der Messeinheit 27 des kombinierten Bildgebungsgeräts 10 aufgenommen.

Im Anschluss an den Verfahrensschritt 51 erfolgt in einem weiteren Verfahrensschritt 53 eine Erstellung von MR-Übersichtsbilddaten auf Basis der Messdaten der MR-Übersichtsmessung mittels der Recheneinheit 24. Zeitgleich zum Verfahrensschritt 53 werden im Verfahrensschritt 54 PET-Übersichtsbilddaten aus den Messdaten der PET-Übersichtsmessung mittels der Recheneinheit 24 erstellt. Die MR-Übersichtsbilddaten sowie die PET-Übersichtsbilddaten können eine grobe Auflösung aufweisen, lassen sich jedoch zeiteffizient erstellen und enthalten sowohl funktionelle als auch strukturelle Informationen über den Patienten 15.

Nach Abschluss der beiden Verfahrensschritte 53 und 54 werden die MR-Übersichtsbilddaten sowie die PET-Übersichtsbilddaten mittels der Anzeigeeinheit 25 für den Benutzer des kombinierten Bildgebungsgeräts 10 visualisiert. In der vorliegenden Ausführungsform des erfindungsgemäßen Verfahrens werden die PET-Übersichtsbilddaten außerdem von einer Planungseinheit 32 in einem weiteren Verfahrensschritt 55 auf pathologische Strukturen hin untersucht. Bei dieser Untersuchung werden nicht näher dargestellte Bildverarbeitungsalgorithmen eingesetzt, welche pathologische Strukturen im Patient 15 erkennen und Bildgebungsbereiche sowie Bildgebungsparameter für die MR-Vorrichtung 11 und die PET-Vorrichtung 12 vorschlagen, mit denen die pathologische Struktur abgebildet werden kann. Der Bildgebungsbereich sowie die Bildgebungsparameter werden dem Benutzer des kombinierten Bildgebungsgeräts mittels der Anzeigeeinheit 25 dargestellt. Um die Planung der Messung der MR-Vorrichtung 11 und der PET-Vorrichtung 12 abzuschließen, kann der Benutzer des kombinierten Bildgebungsgeräts 10 den vorgeschlagenen Bildgebungsbereich sowie die vorgeschlagenen Bildgebungsparameter mittels der Eingabeeinheit 26 bestätigen oder diese manuell auf Basis der visualisierten MR-Übersichtsbilddaten und PET-Übersichtsbilddaten festlegen. Mit der Eingabe des Benutzers wird die Planung der MR-Messung und der PET-Messung abgeschlossen.

In einem anschließenden Verfahrensschritt 56 erfolgt eine Messung des Patienten 15 mittels der MR-Vorrichtung 11 und der Messeinheit 27. Zur Aufnahme der Messung werden der Bildgebungsbereich sowie die Bildgebungsparameter für die MR-Vorrichtung 11 aus dem Verfahrensschritt 55 übernommen. Zeitgleich zur Messung mit der MR-Vorrichtung 11 erfolgt eine Messung des Patienten 15 mit der PET-Vorrichtung 12 und der Messeinheit 27 in einem Verfahrensschritt 57. Auch für die PET-Messung werden der Bildgebungsbereich sowie die Bildgebungsparameter für die PET-Vorrichtung 12 aus dem Verfahrensschritt 55 übernommen. Die Messung mit der MR-Vorrichtung 11 und der PET-Vorrichtung 12 wird jeweils von der Messeinheit 27 koordiniert.

In einem weiteren Verfahrensschritt 58 werden mittels der Messdaten aus Verfahrensschritt 56 und der Recheneinheit 24 Bilddaten der MR-Messung 56 erstellt. Zeitgleich erfolgt eine Erstellung von Bilddaten der PET-Messung 57 mittels der Recheneinheit 24 und der Messdaten aus Verfahrensschritt 57. Die Bilddaten der MR-Messung 56 und der PET-Messung 57 stellen hochaufgelöste Bilddaten relevanter physiologischer und pathologischer Strukturen bereit, welche für eine diagnostische Interpretation durch eine fachkundige Person herangezogen werden können.

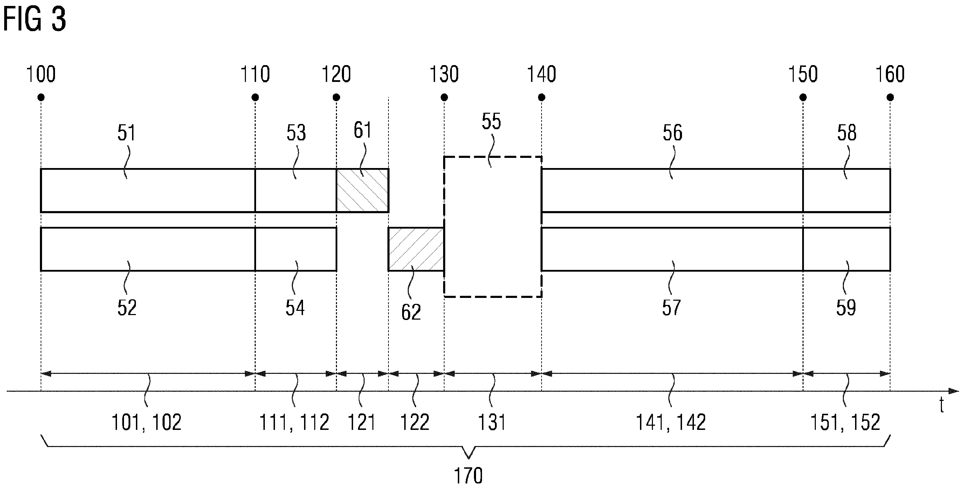

Fig. 3 zeigt einen zeitlichen Ablauf einer bevorzugten Ausführungsform des erfindungsgemäßen Verfahrens, bei welchem anhand der MR-Übersichtsbilddaten eine Schwächungskarte erstellt wird, die zur Schwächungskorrektur der PET-Übersichtsbilddaten verwendet wird. Die dargestellten Zeiträume für die einzelnen Verfahrensschritte sind dabei lediglich als Veranschaulichung des Verfahrensablaufs zu verstehen und können sich bei einer realen Durchführung des Verfahrens deutlich unterscheiden. In der dargestellten Ausführungsform beginnen die MR-Übersichtsmessung 51 und die PET-Übersichtsmessung 52 zum Startzeitpunkt der Untersuchung 100. Die MR-Übersichtsmessung weist dabei eine Messdauer der ersten Übersichtsmessung 101 auf, während die PET-Übersichtsmessung eine Messdauer der zweiten Übersichtsmessung 102 aufweist. Die Messdauer der ersten Übersichtsmessung und die Messdauer der zweiten Übersichtsmessung stimmen in der dargestellten Ausführungsform des erfindungsgemäßen Verfahrens überein, sodass die MR-Übersichtsmessung 51 sowie die PET-Übersichtsmessung 52 zum Endzeitpunkt der Aufnahme der Übersichtsmessung 110 abgeschlossen sind. In dem dargestellten Zeitraum der Messdauer der ersten Übersichtsmessung bewegt sich die Patientenlagerungsvorrichtung 16 kontinuierlich und beispielsweise mehrfach in abwechselnder Richtung durch den Patientenaufnahmebereich 14. Die aufgenommenen Messdaten der PET-Vorrichtung 12, aber auch der MRT-Vorrichtung 11, werden dabei jeweils ihrem gegenüber der kontinuierlichen Bewegung der Patientenlagerungsvorrichtung invarianten Ursprung im Patienten 15 zugeordnet.

Im Anschluss an den Endzeitpunkt der Übersichtsmessung 110 erfolgt die Erstellung der MR-Übersichtsbilddaten 53 und der PET-Übersichtsbilddaten 54 nach dem oben beschriebenen Verfahren. Die Dauer der Erstellung der ersten Übersichtsbilddaten 111 entspricht dabei der Dauer der Erstellung der zweiten Übersichtsbilddaten 112, sodass zum Endzeitpunkt der Erstellung der Übersichtsbilddaten 120 sowohl MR-Übersichtsbilddaten, als auch PET-Übersichtsbilddaten des Patienten 15 vorliegen.

Auf Basis der MR-Übersichtsbilddaten 63 wird anschließend mittels der Recheneinheit 24 eine Schwächungskarte erstellt, welche eine Schwächung von Photonen im Gewebe des Patienten kompensiert. Das Erstellen der Schwächungskarte 61 benötigt eine Dauer der Erstellung der Schwächungskarte 121, in der im aufgeführten Beispiel keine weiteren Messungen durch die Messeinheit 27 erfolgen. Nach dem Erstellen der Schwächungskarte 61 schließt sich die Durchführung der Schwächungskorrektur 62 an, welche durch eine Dauer der Durchführung der Schwächungskorrektur 122 gekennzeichnet ist. Nach Abschluss der Schwächungskorrektur liegen schwächungskorrigierte PET-Übersichtsbilddaten vor, welche zur Planung der Messung 55 herangezogen werden.

Mit dem Startzeitpunkt der Planung der Messung 130 werden auf Basis der PET-Übersichtsbilddaten 64 der Bildgebungsbereich sowie die Bildgebungsparameter der geplanten Messung wie oben beschrieben festgelegt. Während der Planung der Messung 55 werden die MR-Übersichtsbilddaten 63 sowie die PET-Übersichtsbilddaten 64 auf der Anzeigeeinheit 24 für den Benutzer des kombinierten Bildgebungsgeräts 10 visualisiert. Der Benutzer kann während der Dauer der Planung der Messung 131 Einfluss auf die Planung der Messung 55 ausüben, z. B. indem er den Bildgebungsbereich oder die Bildgebungsparameter für die geplante Messung einstellt.

Nach Ablauf der Dauer der Planung der Messung 131 schließt sich der Startzeitpunkt der Messung 140 an. Während der Messdauer des ersten Bildgebungsverfahrens 141 wird die MR-Messung 56 des Patienten 15 durchgeführt. Neben der MR-Messung 56 wird zeitgleich die PET-Messung 57 durchgeführt, welche durch eine Messdauer des zweiten Bildgebungsverfahrens 142 gekennzeichnet ist. Im gezeigten Beispiel stimmt die Messdauer des ersten Bildgebungsverfahrens 141 mit der Messdauer des zweiten Bildgebungsverfahrens 142 überein. Dadurch ergibt sich ein gemeinsamer Endzeitpunkt der Messung 150, an dem die Aufnahme der MR-Messung 56 und der PET-Messung 57 abgeschlossen ist. In dem dargestellten Zeitraum der Messdauer des ersten Bildgebungsverfahrens 141 bewegt sich die Patientenlagerungsvorrichtung 16 kontinuierlich und mehrfach in abwechselnder Richtung durch den Patientenaufnahmebereich 14. Die aufgenommenen Messdaten der PET-Messung 57 und der MR-Messung 56 werden dabei jeweils ihrem gegenüber der kontinuierlichen Bewegung der Patientenlagerungsvorrichtung invarianten Ursprung im Patienten 15 zugeordnet.

Die aufgenommenen Daten der MR-Messung 56 und der PET-Messung 57 werden anschließend zur Erstellung von Bilddaten herangezogen. Das Erstellen der MR-Bilddaten 58 ist durch eine Dauer der Erstellung der ersten Bilddaten 151 gekennzeichnet. Das Erstellen der PET-Bilddaten 59 wird dabei zeitgleich zum Erstellen der MR-Bilddaten 58 mittels der Recheneinheit 24 durchgeführt. Für die Erstellung der PET-Bilddaten wird eine Dauer der Erstellung der zweiten Bilddaten 152 benötigt, welche im aufgeführten Beispiel mit der Dauer der Erstellung der ersten Bilddaten 151 übereinstimmt. Das Erstellen der MR-Bilddaten 58 sowie das Erstellen der PET-Bilddaten 59 wird zum Endzeitpunkt der Untersuchung 160 abgeschlossen. Zu diesem Zeitpunkt werden die MR-Bilddaten sowie die PET-Bilddaten auf der Anzeigeeinheit 25 für den Benutzer des kombinierten Bildgebungsgeräts 10 dargestellt. Eine fachkundige Person kann die MR-Bilddaten sowie die PET-Bilddaten für eine diagnostische Interpretation relevanter pathologischer Regionen des Patienten 15 heranziehen. Das in Fig. 3 gezeigte erfindungsgemäße Verfahren ist durch eine Untersuchungsdauer 170 gekennzeichnet, welche die benötigte Zeit für die gesamte Untersuchung zwischen dem Startzeitpunkt der Untersuchung 100 und dem Endzeitpunkt der Untersuchung 160 angibt.

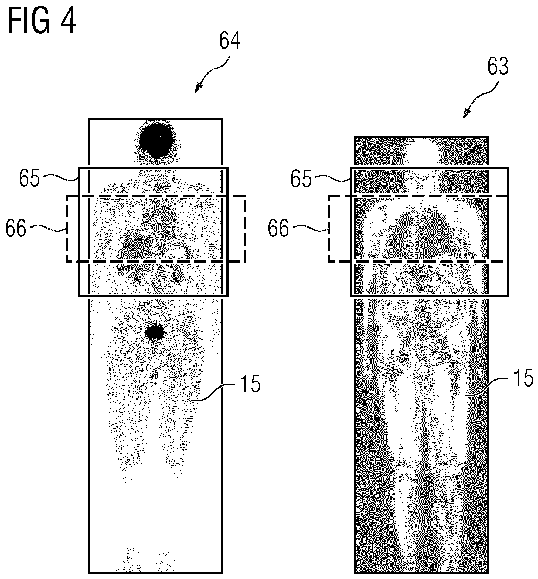

In Fig. 4 sind nebeneinander PET-Übersichtsbilddaten 64 und MR-Übersichtsbilddaten 63 nach dem erfindungsgemäßen Verfahren dargestellt. Die PET-Übersichtsbilddaten 64 ermöglichen eine Darstellung der funktionell aktiven Regionen des Patienten 15, während die MR-Übersichtsbilddaten 63 eine Abbildung der strukturellen Voraussetzungen des Patienten 15 ermöglichen. In den PET-Übersichtsbilddaten 64 kann eine erhöhte funktionelle Aktivität in der Blase sowie im Gehirn des Patienten 15 festgestellt werden, welche auf einer physiologischen Ansammlung der radioaktiven Tracer-Substanz in den entsprechenden Körperregionen beruht. Daneben werden auch erhöhte funktionelle Aktivitäten in der Lunge sowie den Nieren des Patienten 15 vermutet. Diese Tracer-Aktivität weist auf pathologische Strukturen hin, welche bei der anschließenden PET-Messung 57 berücksichtigt werden sollen. Bei der Planung der Messung wird daher ein PET-Bildgebungsbereich 66 sowie ein MR-Bildgebungsbereich 65 auf den Brustraum des Patienten 15 fokussiert.

Im Vergleich zum MR-Bildgebungsbereich 65 weist der PET-Bildgebungsbereich 66 eine geringere Abmessung in Längsrichtung zum Patientenaufnahmebereich 14 auf. Dafür ist der PET-Bildgebungsbereich 66 quer zur Längsrichtung des Patientenaufnahmebereichs größer und deckt auch die oberen Extremitäten des Patienten 15 ab. Bei den MR-Übersichtsbilddaten 63 sind die oberen Extremitäten des Patienten 15 aufgrund des kleineren Bildgebungsbereiches quer zur Längsrichtung des Patientenaufnahmebereichs 14 nicht vollständig abgebildet und können somit nicht für eine Erstellung einer Schwächungskarte herangezogen werden. Werden pathologischen Strukturen in diesen Regionen vermutet, ist die Durchführung einer dritten Übersichtsmessung mittels der MRT-Vorrichtung 11 und der Messeinheit 27 nach dem oben beschriebenen Verfahren erwägenswert, um die außerhalb des üblichen Bildgebungsbereiches liegenden Körperregionen darzustellen und bei der Schwächungskorrektur der PET-Übersichtsbilddaten 64 zu berücksichtigen.

Die MR-Übersichtsbilddaten 63 enthalten gegenüber den PET-Übersichtsbilddaten 64 zusätzliche Informationen zu den strukturellen Vorrausetzungen des Patienten 15 und unterstützen den Benutzer bei der Analyse der Übersichtsbilddaten. Dies ist besonders relevant, wenn Regionen mit geringer funktioneller Aktivität, wie z. B. die unteren Extremitäten des Patienten 15 in den PET-Übersichtsbilddaten 64 der Fig. 4, eine geringe Sichtbarkeit aufweisen.

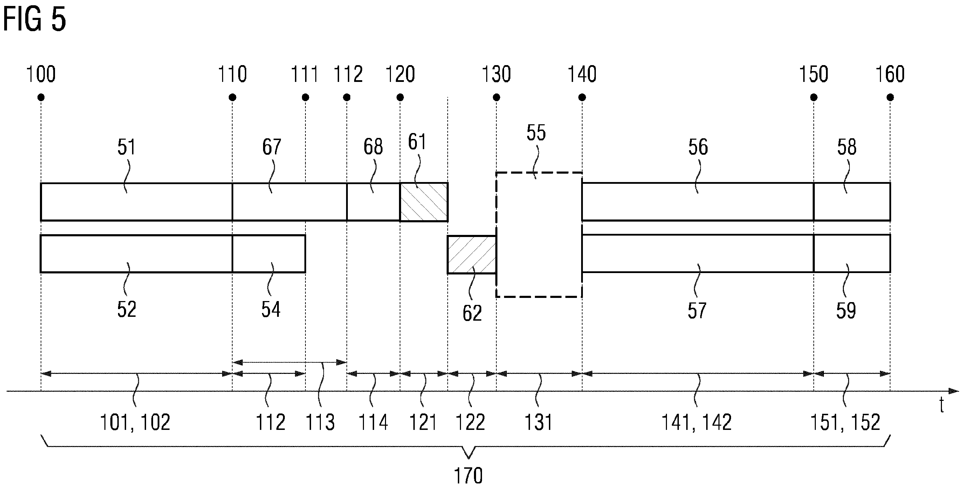

Fig. 5 zeigt einen zeitlichen Ablauf einer Ausführungsform des erfindungsgemäßen Verfahrens, bei welchem eine dritte Übersichtsmessung 67 durch die MRT-Vorrichtung 11 durchgeführt wird. Aus der dritten Übersichtsmessung und der ersten Übersichtsmessung werden anschließend erweiterte erste Übersichtsbilddaten erstellt, die mit den zweiten Übersichtsbilddaten zu kombinierten Übersichtsbilddaten kombiniert werden. Der zeitliche Ablauf des Verfahrens stimmt bis auf den Zeitraum zwischen dem Endzeitpunkt der Aufnahme der Übersichtsmessungen 110 und dem Startzeitpunkt der Messung 140 mit dem in Fig. 3 gezeigten Verfahrensablauf überein. Im Gegensatz zu Fig. 3 schließt sich an den Endzeitpunkt der Aufnahme der Übersichtsmessungen 110 eine dritte Übersichtsmessung 67 mit der MRT-Vorrichtung 11 an. Hierbei wird ein gegenüber der ersten Übersichtsmessung vergrößerter Bildgebungsbereich ("HUGE scan") verwendet, welcher auch die üblicherweise au-ßerhalb des Bildgebungsbereichs liegenden Regionen des Patienten 15 erfasst.

Nach Ablauf der Messdauer der dritten Übersichtsmessung 113 erfolgt ein Erstellen von erweiterten ersten Übersichtsbilddaten 68 anhand der Messdaten der ersten Übersichtsmessung 51 und den Messdaten der dritten Übersichtsmessung 67. Nach Ablauf der Dauer der Erstellung der erweiterten ersten Übersichtsbilddaten 114 schließt sich ein Erstellen der Schwächungskarte 61 auf Basis der erweiterten ersten Übersichtsbilddaten an. Die so erstellte Schwächungskarte wird wie oben beschrieben zur Schwächungskorrektur der zweiten Übersichtsbilddaten eingesetzt und berücksichtigt auch die üblicherweise außerhalb des Bildgebungsbereichs des ersten Bildgebungsverfahrens lokalisierten Körperregionen des Patienten 15.

Im Gegensatz zum Ablauf des Verfahrens in Fig. 3 werden in der vorliegenden Ausführungsform zum Startzeitpunkt der Planung der Messung 130 kombinierte Übersichtsbilddaten aus den erweiterten ersten Übersichtsbilddaten und den zweiten Übersichtsbilddaten durch die Recheneinheit 24 erstellt. Noch während des Zeitraums der Planung der Messung 55 werden die kombinierten Übersichtsbilddaten auf der Anzeigeeinheit 25 für den Benutzer des kombinierten Bildgebungsgeräts 10 visualisiert und von der Planungseinheit 32 zur Planung der Messung des ersten Bildgebungsverfahrens und des zweiten Bildgebungsverfahrens herangezogen. Ab dem Startzeitpunkt der Messung 140 stimmt die Ausführungsform in Fig. 5 wieder mit dem zeitlichen Ablauf der Ausführungsform in Fig. 3 überein.

Obwohl die Erfindung im Detail durch die bevorzugten Ausführungsbeispiele näher illustriert und beschrieben wurde, so ist die Erfindung dennoch nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zur Erstellung von Bilddaten eines Untersuchungsobjekts unter Verwendung eines kombinierten Bildgebungsgeräts, welches ein erstes Bildgebungsverfahren und ein zweites Bildgebungsverfahren aufweist, wobei das erste Bildgebungsverfahren eine strukturelle Bildgebung mittels eines Magnetresonanzgerätes aufweist und das zweite Bildgebungsverfahren eine funktionelle Bildgebung aufweist, mit den folgenden Schritten:
• Aufnahme einer ersten Übersichtsmessung mittels des ersten Bildgebungsverfahrens und einer zweiten Übersichtsmessung mittels des zweiten Bildgebungsverfahrens, wobei sich die erste Übersichtsmessung zeitlich mit der zweiten Übersichtsmessung überschneidet;
• Erstellen von ersten Übersichtsbilddaten basierend auf der ersten Übersichtsmessung und von zweiten Übersichtsbilddaten basierend auf der zweiten Übersichtsmessung;
• Planung einer Messung des ersten Bildgebungsverfahrens und des zweiten Bildgebungsverfahrens auf Basis der ersten Übersichtbilddaten und der zweiten Übersichtsbilddaten;
• Durchführung der geplanten Messung des ersten Bildgebungsverfahrens und des zweiten Bildgebungsverfahrens;
• Erstellen der Bilddaten des Untersuchungsobjektes auf Basis der Messung des ersten Bildgebungsverfahrens und des zweiten Bildgebungsverfahrens.

2. Verfahren nach Anspruch 1, wobei die erste Übersichtsmessung und die zweite Übersichtsmessung bei einer kontinuierlichen Bewegung einer Patientenlagerungsvorrichtung des kombinierten Bildgebungsgeräts erfolgen.

3. Verfahren nach Anspruch 2, wobei das zweite Bildgebungsverfahren eine Positronen-Emissions-Tomographie umfasst, wobei eine Anzahl von Annihilierungsereignissen bei der kontinuierlichen Bewegung der Patientenlagerungsvorrichtung mittels eines Detektors zeitabhängig registriert und als zeitabhängiger Wert abgespeichert werden.

4. Verfahren nach Anspruch 3, wobei die Aufnahme der zweiten Übersichtsmessung umfasst:
• Zuordnen der registrierten Annihilierungsereignisse einem gegenüber der kontinuierlichen Bewegung der Patientenlagerungsvorrichtung invarianten Ursprung im Untersuchungsobjekt, wobei die kontinuierliche Bewegung der Patientenlagerungsvorrichtung berücksichtigt wird,
• Abspeichern der zugeordneten, registrierten Annihilierungsereignisse als zweite Übersichtsmessung.

5. Verfahren nach einem der vorhergehenden Ansprüche, wobei auf Basis der ersten Übersichtsbilddaten eine Schwächungskarte des Untersuchungsobjektes erstellt wird, wobei anhand der Schwächungskarte eine Schwächungskorrektur der zweiten Übersichtsbilddaten erfolgt, wobei die Planung der Messung auf Basis der schwächungskorrigierten zweiten Übersichtsbilddaten erfolgt.

6. Verfahren nach den Ansprüchen 4 und 5, wobei auf Basis der bei kontinuierlicher Bewegung der Patientenlagerungsvorrichtung aufgenommenen ersten Übersichtsmessung eine Schwächungskarte erstellt wird, wobei die Schwächungskarte zur Schwächungskorrektur der zweiten Übersichtsbilddaten, welche ihrem gegenüber der kontinuierlichen Bewegung der Patientenlagerungsvorrichtung invarianten Ursprung im Untersuchungsobjekt zugeordnete Annihilierungsereignisse enthalten, erfolgt.

7. Verfahren nach einem der vorhergehenden Ansprüche, wobei nach der ersten Übersichtsmessung eine dritte Übersichtsmessung mit einem gegenüber der ersten Übersichtsmessung erweitertem Bildgebungsbereich mittels des ersten Bildgebungsverfahrens durchgeführt wird, wobei anhand der ersten Übersichtsmessung und der dritten Übersichtsmessung erweiterte erste Übersichtsbilddaten erstellt werden.

8. Verfahren nach Anspruch 7, wobei die Schwächungskarte des Untersuchungsobjektes anhand der erweiterten ersten Übersichtsbilddaten erstellt wird, wobei auf Basis der Schwächungskarte eine Schwächungskorrektur der zweiten Übersichtsbilddaten erfolgt, wobei die Planung der Messung auf Basis der schwächungskorrigierten zweiten Übersichtsbilddaten erfolgt.

9. Verfahren nach einem der vorhergehenden Ansprüche, wobei auf Basis der ersten Übersichtsbilddaten und der zweiten Übersichtsbilddaten kombinierte Übersichtsbilddaten erstellt und zur Planung der Messung herangezogen werden.

10. Verfahren nach einem der vorhergehenden Ansprüche, wobei die zweiten Übersichtsbilddaten gegenüber den ersten Übersichtsbilddaten Informationen über Regionen mit erhöhter funktioneller Aktivität im Untersuchungsobjekt aufweisen, wobei die Planung der Messung auf Basis dieser Regionen mit erhöhter funktioneller Aktivität erfolgt.

11. Verfahren nach den Ansprüchen 9 und 10, wobei die kombinierten Übersichtsbilddaten gegenüber den ersten Übersichtsbilddaten und den zweiten Übersichtsbilddaten erweiterte Informationen zu pathologischen Strukturen und ihren Positionen im Untersuchungsobjekt aufweisen, wobei die kombinierten Übersichtsbilddaten auf pathologische Strukturen hin untersucht werden und die Planung der Messung auf Basis der identifizierten pathologischen Strukturen erfolgt.

12. Kombiniertes Bildgebungsgerät, umfassend eine Messeinheit, wobei das kombinierte Bildgebungsgerät zur Durchführung des Verfahrens nach einem der vorangegangenen Ansprüche ausgebildet ist.

13. Computerprogrammprodukt, welches direkt in einen Speicher einer programmierbaren Recheneinheit eines kombinierten Bildgebungsgeräts ladbar ist, mit Programmcode-Mitteln, um ein Verfahren nach einem der Ansprüche 1-11 auszuführen, wenn das Computerprogrammprodukt in der Recheneinheit des kombinierten Bildgebungsgeräts ausgeführt wird.
